# EUROPEAN PATENT APPLICATION

(11) **EP 3 988 024 A1**
(43) Date of publication of application: **27.04.2022**
(21) Application number: 20833577.8
(22) Date of filing: 09.06.2020
(51) Int. Cl.: A61B 5/28

(54) **BIOELECTRODE PROVIDED WITH ELECTRONIC CIRCUIT BOARD**

(30) Priority: 24.06.2019 JP 2019116100
(71) Applicant: I Medex Co., Ltd., Chiba-shi, Chiba 262-0003 (JP)
(72) Inventor: ICHIDA Shinshichi, Chiba-shi Chiba 262-0003 (JP); ICHIDA Makoto, Chiba-shi Chiba 262-0003 (JP); MINOWA Takashiro, Chiba-shi Chiba 262-0003 (JP); NAGAHAMA Norio, Chiba-shi Chiba 262-0003 (JP)
(74) Representative: Lavoix
(86) International application number: PCT/JP2020/022709
(87) International publication number: WO 2020/261981

(57) **Abstract**

An object of the present invention is to manufacture, desirably at a low cost, a bioelectrode (10) including various electronic circuit boards (20) and/or electronic components (23), in which an electrode portion (13) and/or a wiring portion (12, 22) is provided on a flexible (pliable) base material (16). The bioelectrode (10) is a bioelectrode including a film-like or sheet-like flexible base material (16) and one or more electrode portions (13) and/or wiring portions (12, 22) provided on the base material (16), and includes the electronic circuit board (20) and/or the electronic component (23) on the base material (16), in which a circuit of the electronic circuit board (20) and/or a connection portion of the electronic component (23) is electrically connected to at least any one electrode portion (13) and/or wiring portion (12, 22) provided on the base material (16).

## Description

### Technical Field

The present invention relates to a bioelectrode including a film-like or sheet-like flexible base material and a wiring provided on the base material, and more particularly, to a bioelectrode including an electronic circuit board and/or an electronic component provided on the base material.

### Background Art

Conventionally, a device that extracts an electric signal from a living body and performs a treatment, and a device that extracts, from a living body, and measures an electric signal, such as an electrocardiograph, an electroencephalograph, or an electromyograph have been known. A bioelectrode is used in these devices. In addition, such a bioelectrode is also used to introduce an electric signal such as a weak current with a low frequency or electromagnetic waves with a high frequency into the body. That is, the bioelectrode is also used for promoting blood circulation, obtaining a massage effect, or the like, or for an iontophoresis treatment in which a drug is subjected to iontophoresis and introduced into the body through the skin.

Conventionally, bioelectrodes including a measurement amplifier, an electric circuit, or the like have also been proposed. For example, Patent Literature 1 (JP H7-108039 A) proposes a body information detection device that detects a potential between two points of a body and processes the detected signal, in which a detection electrode is disposed on a printed circuit board, an instrumentation amplifier is disposed on the printed circuit board, the detection electrode is connected to an input of the instrumentation amplifier, and an output of the instrumentation amplifier is applied as a detection signal.

Conventionally, a paste-like or film-like anisotropic conductive material used to obtain various connection structures has also been known. Such an anisotropic conductive material has a plurality of conductive particles dispersed in a binder resin, and is used for connection between a flexible printed board and a glass board, connection between a semiconductor chip and a flexible printed board, and the like.

For example, Patent Literature 2 (JP 2015-80601 A) proposes a pulse wave sensor using the paste-like or film-like anisotropic conductive material and a biological information measurement device using the pulse wave sensor. In the pulse wave sensor and the biological information measurement device using the pulse wave sensor, reflected light detection element is mounted on a first main surface of a sensor board using an anisotropic conductive film or an anisotropic conductive paste in the pulse wave sensor that optically measures a pulse wave of a subject, the biological information measurement device using the pulse wave sensor, and the like. Patent Literature 2 discloses that, for example, a glass epoxy board can be used as the sensor board.

### Citation List

### Patent Literature

Patent Literature 1: JP H7-108039 A
Patent Literature 2: JP 2015-80601 A

### Summary of Invention

### Technical Problem

As described above, some bioelectrodes including a measurement amplifier, an electric circuit, or the like have been previously proposed. However, electrical connection of the measurement amplifier, the electric circuit, and the like has been made by a conventional method such as soldering. In addition, a biological information measurement device using a pulse wave sensor provided using an anisotropic conductive film or an anisotropic conductive paste has also been proposed, but a sensor board including a pulsation sensor is a glass epoxy board and does not have flexibility.

Meanwhile, in recent years, a bioelectrode, in which an electrode portion and/or a wiring portion is provided on a film-like or sheet-like flexible base material by printing or the like, has been provided. It is difficult to perform connection processing on such a bioelectrode by soldering due to physical properties of the base material and the wiring, and thus it is practically impossible to provide an electronic circuit board or an electronic component on the bioelectrode.

Thus, an object of the present invention is to provide a bioelectrode including various electronic circuit boards and/or electronic components, in which an electrode portion and/or a wiring portion is provided on a film-like or sheet-like flexible (pliable) base material.

Furthermore, another object of the present invention is to provide a bioelectrode that can be manufactured with low cost, the bioelectrode including various electronic circuit boards and/or electronic components while using a film-like or sheet-like flexible (pliable) base material.

### Solution to Problem

In order to solve at least any one of the above problems, the present invention provides a bioelectrode including various electronic circuit boards and/or electronic components electrically connected to wiring, in which an electrode portion and/or a wiring portion is provided on a film-like or sheet-like flexible base material.

That is, the present invention provides a bioelectrode including an electronic circuit board, the bioelectrode including: a film-like or sheet-like flexible base material; and one or more electrode portions and/or wiring portions provided on the base material, in which a circuit of the electronic circuit board and a connection portion of an electronic component is electrically connected to at least any one electrode portion and/or wiring portion provided on the base material by using an anisotropic conductive paste material or anisotropic conductive film material.

Further, the present invention provides a bioelectrode including an electronic circuit board, the bioelectrode including: a film-like or sheet-like flexible base material; and one or more electrode portions and/or wiring portions provided on the base material, in which the electronic circuit board formed separately from the base material is provided on the base material, and at least any one electrode portion and/or wiring portion provided on the base material and a circuit provided on the electronic circuit board are electrically connected by an anisotropic conductive paste material or anisotropic conductive film material.

It is desirable to use a component or material capable of selectively connecting only intended wirings and terminals for connection between at least any one electrode portion and/or wiring portion provided on the base material and the circuit provided on the electronic circuit board and/or the terminal of the electronic component. For example, it is desirable to use the anisotropic conductive paste material or anisotropic conductive film material as the connection component or material. Further, the electronic circuit board may be either a flexible circuit board or a rigid circuit board, and one or more electronic circuit boards and/or electronic components may be provided on the base material.

### Advantageous Effects of Invention

With the bioelectrode according to the present invention, it is possible to electrically connect the circuit provided on the electronic circuit board to at least any one electrode portion and/or wiring portion provided on the base material even in the bioelectrode using the film-like or sheet-like flexible base material. As a result, it is possible to provide a flexible (pliable) bioelectrode including various electronic circuit boards while using the base material.

In addition, according to the present invention, in the bioelectrode that transmits or receives an electric signal acquired from a living body, an electric signal for performing a low-frequency treatment on a body part, or an electric signal for performing an iontophoresis treatment, the electric signal can pass through an electronic circuit or an electronic component.

According to the present invention, the separately formed electronic circuit board can be electrically connected to the film-shaped or sheet-shaped flexible (pliable) base material. As a result, the base material on which the electrode portion and/or the wiring portion is provided and the electronic circuit board can be manufactured in separate processes. When the base material and the electronic circuit board are electrically connected by using the anisotropic conductive paste material or the anisotropic conductive film material, the manufacturing cost and the like can be reduced, and it is possible to manufacture the bioelectrode at a low cost.

### Brief Description of Drawings

Fig. 1(A) is a front view illustrating a bioelectrode according to a first embodiment, Fig. 1(B) is a plan view illustrating the bioelectrode according to the first embodiment, and Fig. 1(C) is an exploded perspective view illustrating the bioelectrode according to the first embodiment.
Fig. 2(A) is an enlarged front view of an electronic circuit board portion in the bioelectrode according to the first embodiment, and Fig. 2(B) is a cross-sectional view taken along line X-X.
Fig. 3(A) is a front view illustrating a bioelectrode according to a second embodiment, Fig. 3(B) is a plan view illustrating the bioelectrode according to the second embodiment, Fig. 3(C) is an exploded perspective view illustrating the bioelectrode according to the second embodiment, and Fig. 3(D) is a cross-sectional view taken along like X-X.
Fig. 4(A) is a front view illustrating a bioelectrode according to a third embodiment, and Fig. 4(B) is an exploded perspective view illustrating the bioelectrode according to the third embodiment.
Fig. 5(A) is a front view illustrating a bioelectrode according to a fourth embodiment, and Fig. 5(B) is an exploded perspective view illustrating the bioelectrode according to the fourth embodiment.
Fig. 6(A) is a front view illustrating a bioelectrode according to a fifth embodiment, and Fig. 6(B) is an exploded perspective view illustrating the bioelectrode according to the fifth embodiment.

### Description of Embodiments

Hereinafter, a bioelectrode 10 according to the present embodiment will be described in detail with reference to the drawings. In particular, the present embodiment illustrates a specific example of the bioelectrode 10 on which an electronic circuit board 20 is mounted and which acquires an electric signal from a living body. However, the present invention is not limited thereto, and can be embodied as, for example, a bioelectrode for performing a low-frequency treatment or a high-frequency treatment on a body part or a bioelectrode for performing an iontophoresis treatment.

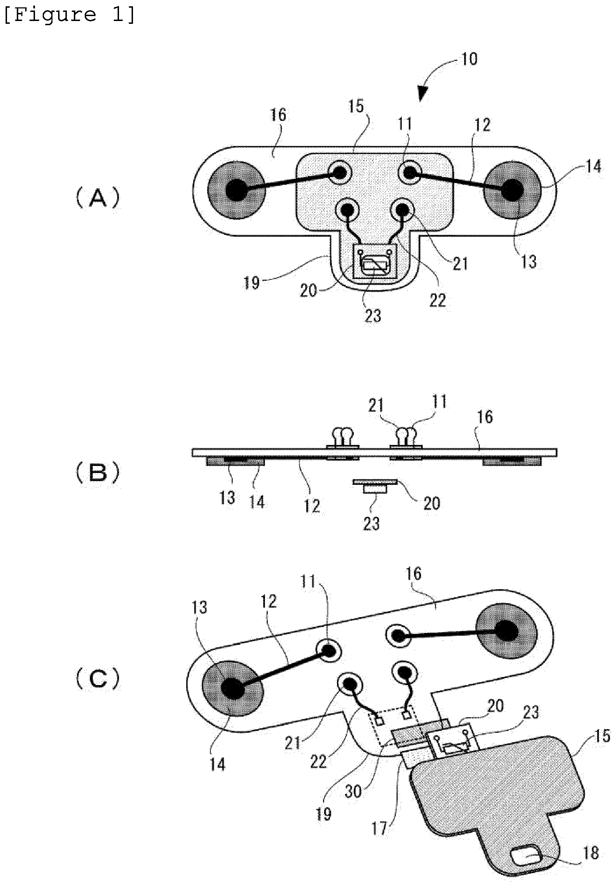

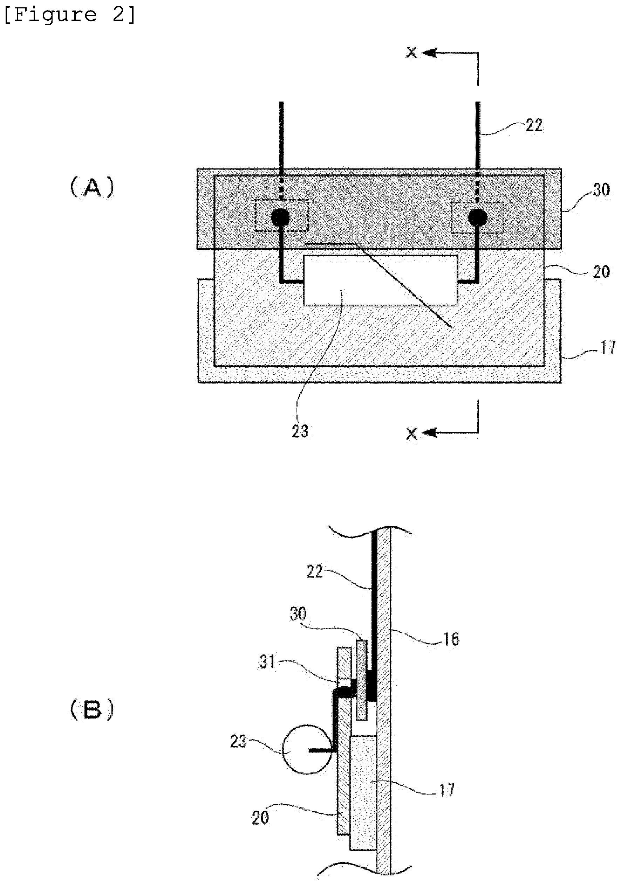

The bioelectrode 10 according to a first embodiment will be described in detail with reference to Figs. 1 and 2. The bioelectrode 10 according to the first embodiment uses a sheet-like or film-like base material 16 that is elongated and has a central portion bulging on one side in a width direction. The base material 16 has flexibility, and can be formed by using a resin sheet or a resin film formed of a resin such as a polyethylene terephthalate resin, a polyethylene naphthalate resin, a polyethylene resin, a polypropylene resin, a polystyrene resin, an acrylonitrile-styrene resin, an acrylonitrile-butadienestyrene resin, a polyvinyl chloride resin, a methacrylic resin, a polycarbonate resin, a polyurethane resin, or a silicone resin, or a composite (multilayer) resin thereof, as well as a fabric such as a nonwoven fabric or a cloth, paper, or the like.

Electrode portions 13 for acquiring a signal from the living body are provided on both end sides of a surface of the base material 16 that faces the living body (hereinafter, referred to as "living-body-side surface") in a length direction. Biological signal terminals 11 corresponding to the electrode portions 13, respectively, are provided at the center of the base material 16. The biological signal wiring portions 12 electrically connect the respective electrode portions 13 and the respective biological signal terminals 11 corresponding thereto. The electrode portion 13 and the biological signal wiring portion 12 can be formed by applying or printing a conductive material such as silver or silver chloride onto the base material 16. In particular, in the present embodiment, both the electrode portion 13 and the biological signal wiring portion 12 are provided on the living-body-side surface of the base material 16. Thus, the electrode portion 13 and the biological signal wiring portion 12 can be formed on the base material 16 by application or printing onto the same surface without inversion of the base material 16. As the biological signal terminal 11, a terminal corresponding to a terminal structure of a device or a wiring connected to the bioelectrode 10 is used.

The electronic circuit board 20 on which at least one of an integrated circuit, a sensor, or an amplifier is mounted is provided in a center portion of the living-body-side surface of the base material 16. Specifically, the electronic circuit board 20 is provided in a bulging portion 19 bulging in the width direction and provided in the central portion of the base material 16. A flexible circuit board or a rigid circuit board can be used as the electronic circuit board 20. The electronic circuit board 20 can be bonded to the base material 16 by using an adhesive member 17 such as an adhesive or a double-sided tape.

A board terminal 21 for outputting and inputting an electric signal of a circuit portion mounted on the electronic circuit board 20 is provided on the base material 16. A board wiring portion 22 is connected to the board terminal 21, and the circuit portion mounted on the electronic circuit board 20 is connected to the board wiring. The board wiring portion 22 and the circuit portion provided on the electronic circuit board 20 can be electrically connected by using an anisotropic conductive paste material 30 or an anisotropic conductive film material. Note that, since the board wiring portion 22 is also provided on the living-body-side surface of the base material 16 similarly to the electrode portion 13 and the biological signal wiring portion 12, the board wiring portion 22 can be formed without inverting the base material 16.

In particular, in the present embodiment, the electronic circuit board 20 can be provided on the living-body-side surface of the base material 16. Thus, an electronic component 23 such as a sensor provided on the electronic circuit board 20 can be brought into direct contact with the living body. For example, in a case where the electronic component 23 is a thermistor for detecting a body temperature of the living body, a pH sensor for measuring acidity of the surface of the living body, and the like, these sensors can be brought into direct contact with the surface of the living body, and as a result, measurement accuracy can be improved.

Here, the sensor is not limited to a sensor that comes into contact with the living body, such as the thermistor or the pH sensor. The sensor may include various sensors such as an acceleration sensor and a gyro sensor. The electronic component 23 other than the sensor can also be mounted on the electronic circuit board 20. For example, various electronic components 23 such as an amplifier that amplifies an electric signal, a rectifier that rectifies an electric signal, the electronic component 23 that counts the number of times of energization, and an integrated circuit (read only memory (ROM) or the like) for performing electronic ID, authentication, counterfeit product discrimination, and the like can be mounted. That is, various electronic components 23 known at the time of filing of the present application can be mounted on the electronic circuit board 20.

In the bioelectrode 10 according to the present embodiment, a cover sheet 15 is provided on the living-body-side surface of the base material 16 so as to cover the respective wiring portions, the biological signal terminal 11, and the board terminal 21. The cover member can be formed of a material having electrical non-conductivity (insulation property), and as a result, an electrical short circuit between the respective wiring portions and the terminals can be prevented. The cover sheet 15 can be formed of a material having an insulation property and flexibility to such an extent that followability at the time of attachment can be secured. In addition, in a case where it is desirable that the sensor mounted on the electronic circuit board 20 comes into direct contact with the living body, an opening 18 can be formed in a corresponding region of the cover sheet 15. The sensor or the like mounted on the electronic circuit board 20 can come into direct contact with the living body via the opening 18. Furthermore, the bioelectrode 10 according to the present embodiment can include a resist layer formed of a non-conductive material in order to prevent a short circuit in each wiring portion and the electrode portion 13. In addition, the bioelectrode 10 can also include a shield layer formed of a conductive material in order to prevent mixing of electromagnetic waves or static electricity from the outside.

A gel electrolyte member 14 having conductivity is provided on the living-body-side surface of the base material 16, which is the surface of the electrode portion 13 that faces the living body. The gel electrolyte member 14 comes into contact with the living body and transmits a biological signal to the electrode portion 13 or guides a weak current from the electrode portion 13 to the living body. It is desirable that the gel electrolyte portion has semi-fluidity or fluidity in order to enhance adhesion to the living body.

Next, a connection state between the circuit portion provided on the electronic circuit board 20 and the board wiring portion 22 provided on the base material 16 will be described with reference to Fig. 2. As described above, the electrical connection between the board wiring portion 22 provided on the sheet-like base material 16 and the electronic circuit provided on the electronic circuit board 20 is implemented by the anisotropic conductive paste material 30. The anisotropic conductive paste material 30 is formed by dispersing fine metal particles in a thermosetting resin. The dispersed particles can be brought into contact with each other by thermocompression bonding to form a conductive path at a protrusion such as the wiring portion or the terminal of the circuit portion. On the other hand, the particles present in a region where no pressure is applied at the time of thermocompression bonding (that is, a region where the wiring portion or the terminal of the circuit portion is not present in the base material 16) maintain a state where the particles are dispersed in the resin, and the insulation property can be maintained. Thus, by using the anisotropic conductive paste material 30, anisotropy in which the conductivity is maintained in a longitudinal direction and the insulation property is maintained in a transverse direction can be formed.

In the bioelectrode according to the present embodiment, the electronic circuit board is electrically connected to the electrode portion and/or the wiring portion provided on the board by using the anisotropic conductive paste material 30 (or an anisotropic paste film). As a result, it is possible to manufacture the bioelectrode using the flexible (pliable) base material and including the electronic circuit board at a low cost. That is, a flexible board formed of a polyimide material that enables soldering of various electronic components may be used in order to maintain flexibility (pliability) and mount the electronic component. However, the electrode portion (silver or silver chloride) on which the gel electrolyte member that comes into contact with the living body is provided requires processing such as plating. As a result, an expensive bioelectrode is formed due to the cost of materials such as a flexible board and the complexity of processes, and it is difficult to use the bioelectrode in a disposable manner.

On the other hand, the bioelectrode according to the present embodiment can be manufactured at a low cost by printing the electrode portion and/or the wiring portion on the base material. The electronic circuit board that can be further downsized and has favorable mass productivity can be manufactured in a process separate from the manufacturing of the base material. As a result, the base material can be formed using the above-described various materials, and the electronic circuit board can also be manufactured at a low cost by the existing technology. Thus, it is possible to manufacture the bioelectrode which can be used in a disposable manner and on which the electronic component can be mounted.

The board-side wiring is formed on the living-body-side surface of the base material 16, and the electronic circuit board 20 is also provided on the living-body-side surface of the base material 16. The electronic component 23 is mounted on the electronic circuit board 20. Specifically, the electronic component 23 is mounted on a surface (a living-body-side surface of the electronic circuit board 20) of the electronic circuit board 20 that is opposite to a surface facing the base material 16. Thus, it is necessary to guide the terminal of the circuit portion provided on the electronic circuit board 20 to the surface facing the board wiring portion 22. In the present embodiment, a through-hole 31 is provided in the electronic circuit board 20, and the terminal connected to the circuit portion is guided to the surface facing the board wiring portion 22 through the through-hole 31. That is, electrical connection between the circuit portion provided on the living-body-side surface of the electronic circuit board 20 and the terminal portion provided on the opposite side is implemented by the through-hole 31. The anisotropic conductive paste material 30 is provided between the board-side wiring and a portion where the through-hole 31 of the electronic circuit board 20 is formed. Then, a multilayer structure region of the base material 16, the board wiring portion 22, the anisotropic conductive paste material 30, the through-hole 31, and the electronic circuit board 20 is subjected to thermocompression bonding. As a result, the board wiring portion 22 and the through-hole 31 (the terminal of the circuit portion) can be electrically connected in regions facing each other. In particular, in the bioelectrode 10 according to the present embodiment, the base material 16 is formed in a flexible sheet shape. Thus, in a region where the board wiring portion 22 and the terminal of the circuit portion are not present, a pressure for thermocompression bonding is not applied due to deformation of the base material 16, and the insulation property can be secured. That is, in the bioelectrode 10 according to the present embodiment, since the base material 16 has flexibility, electrical non-conductivity (insulation property) can be secured.

Note that, in the present embodiment, an anisotropic film material can be used instead of the anisotropic conductive paste material 30. In addition, an electrical connection portion between the board wiring portion 22 and the through-hole 31 (the terminal of the circuit portion) of the electronic circuit board 20 can be configured so that a pressure at the time of thermocompression bonding is selectively or preferentially applied to the anisotropic conductive paste material 30 present therebetween. For example, a protrusion can be formed on the electronic circuit board 20, or a jig having a protrusion can be installed under the base material 16 and subjected to thermocompression bonding.

In the present embodiment described above, the electronic component 23 is mounted on the electronic circuit board 20, and the electronic circuit board 20 is provided on the base material 16 of the bioelectrode 10, but the electronic component may be directly provided on the base material 16. That is, the terminal in the electronic component 23 can be electrically connected directly to the board wiring portion 22 of the base material 16 by using the anisotropic conductive paste material 30. In such electrical connection, it is a matter of course that a wiring member is interposed therebetween.

The bioelectrode 10 according to the present embodiment can be manufactured by performing: a conductive material application step of applying or printing the conductive material on the film-like or sheet-like flexible base material 16 to form the electrode portion 13 and/or the wiring portion; a layering step of layering the anisotropic conductive paste material 30 or the anisotropic conductive film material on at least any one electrode portion 13 and/or wiring portion formed by application or printing, and layering the electronic circuit board 20 formed separately from the base material 16 on the anisotropic conductive paste material 30 or the anisotropic conductive film material; and a thermocompression bonding step of electrically connecting the electrode portion 13 and/or the wiring portion, the anisotropic conductive paste material 30 or the anisotropic conductive film material, and the electronic circuit board 20 by thermocompression bonding.

With the bioelectrode 10 configured as described above according to the present embodiment, it is possible to electrically connect the electronic circuit board 20 on which the electronic component 23 that is originally required to be soldered is mounted while using the flexible base material 16. As a result, it is possible to manufacture the bioelectrode 10 on which the electronic component 23 is mounted while using the flexible base material 16.

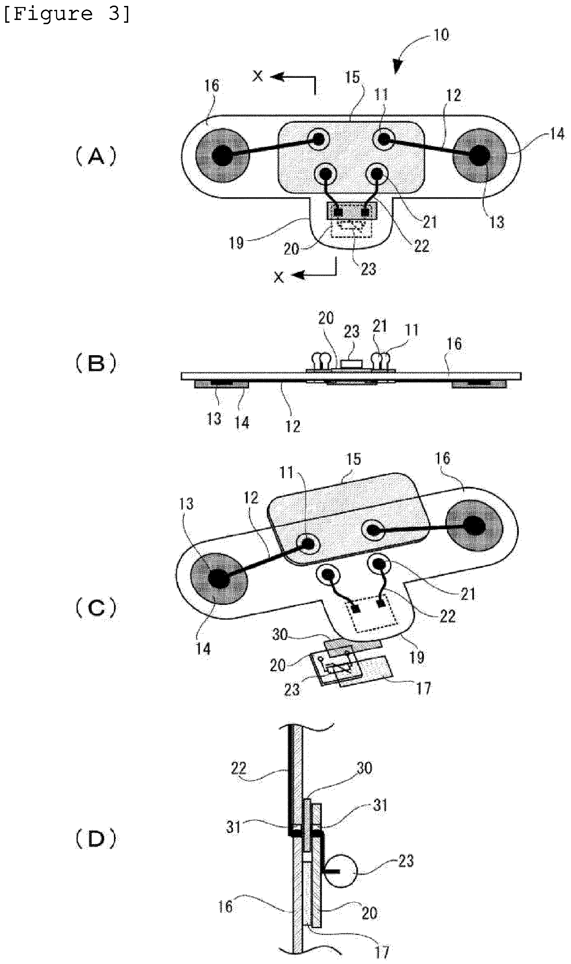

A bioelectrode 10 according to a second embodiment will be described with reference to Fig. 3. In particular, in the bioelectrode 10 according to the present embodiment, an electronic circuit board 20 on which an electronic component 23 is mounted is provided on a surface (hereinafter, referred to as a "back side surface") that is opposite to a living-body-side surface of the base material 16.

That is, the bioelectrode 10 according to the second embodiment uses the base material 16 described in the first embodiment, and the electrode portion 13, the gel electrolyte member 14, the wiring portion, the biological signal terminal 11, the board wiring portion 22, the board terminal 21, and the cover sheet 15 described in the first embodiment are provided on the living-body-side surface of the base material 16. The electronic circuit board 20 on which the electronic component 23 is mounted is provided on the back side of the base material 16.

In the bioelectrode 10 according to the present embodiment, the board wiring portion 22 and the circuit portion of the electronic circuit board 20 are provided on opposite surfaces, respectively. So, a through-hole 31 is provided in the base material 16 in order to electrically connect the board wiring portion 22 and the circuit portion of the electronic circuit board 20. That is, as illustrated in Fig. 3, the through-hole 31 is formed in a portion of the base material 16 that corresponds to the terminal portion of the board wiring portion 22 in order to take the wiring portion provided on the living-body-side of the base material 16 to the back side. An anisotropic conductive paste material 30 is provided at a position where the through-hole 31 is formed, and the terminal portion of the electronic circuit board 20 is overlapped thereon. By performing thermocompression bonding thereon, the through-hole 31 of the base material 16 and the terminal portion of the electronic circuit board 20 can be electrically connected via the anisotropic conductive paste material 30.

In the bioelectrode 10 formed in this manner, the electronic circuit board 20 on which the electronic component 23 is mounted can be provided on the back side surface of the base material 16. Thus, the electronic component 23 mounted on the electronic circuit board 20 is present on an outer surface (that is, a back side surface) of the bioelectrode 10, and even in a case where unevenness occurs due to the mounting of the electronic component 23, it is possible to prevent the unevenness from coming into contact with the living body.

As described above, in the bioelectrode 10 according to the present embodiment, the through-hole 31 is provided in the base material 16, but the through-hole 31 can also be provided in the electronic circuit board 20 depending on an installation orientation (a surface where the terminal portion is present) of the electronic circuit board 20. Thus, in the bioelectrode 10 according to the present embodiment, the through-hole 31 can be provided in at least one of at least any one electrode portion 13 and/or wiring portion provided on the base material 16 or the circuit portion provided on the electronic circuit board 20.

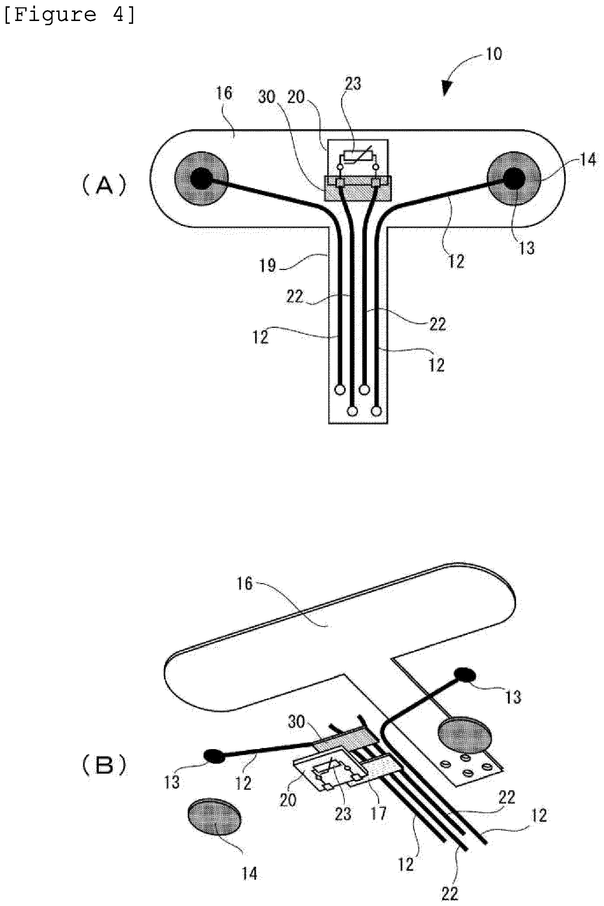

A bioelectrode 10 according to a third embodiment will be described with reference to Fig. 4. In the bioelectrode 10 according to the present embodiment, a shape of a base material 16 and a structure of each terminal portion are different from those of the bioelectrodes 10 according to the first and second embodiments.

That is, the base material 16 is formed in a "T" shape, and a biological signal wiring portion connected to each electrode portion 13 and a board wiring portion 22 connected to an electronic circuit board 20 are provided on an extending portion 19 extending to one side. Then, a connection portion for connecting a biological signal transmission/reception device that acquires an electric signal from the bioelectrode 10 or outputs an electric signal to the bioelectrode 10, or a cable extending from the biological signal transmission/reception device is provided on a distal end side of the electronic circuit board 20. In particular, since the extending portion 19 for providing each wiring portion is provided on the base material 16, the electronic circuit board 20 can be provided at a central portion of the base material 16 formed in the "T" shape, that is, between the electrode portions 13. As a result, the electronic circuit board 20 is present between gel electrolyte members 14 that come into close contact with the living body, and thus can be reliably brought into close contact with the living body. As described in the present embodiment, when various wiring portions and connection portions are formed on the electronic circuit board 20, it is desirable to use a flexible circuit board having flexibility as the base material 16.

Note that, in the bioelectrode 10 according to the present embodiment, the electronic circuit board 20 is provided on a living-body-side surface of the base material 16 as in the bioelectrode 10 according to the first embodiment. However, the bioelectrode 10 according to the present embodiment can also be provided on the back side surface of the base material 16 as in the bioelectrode 10 according to the second embodiment.

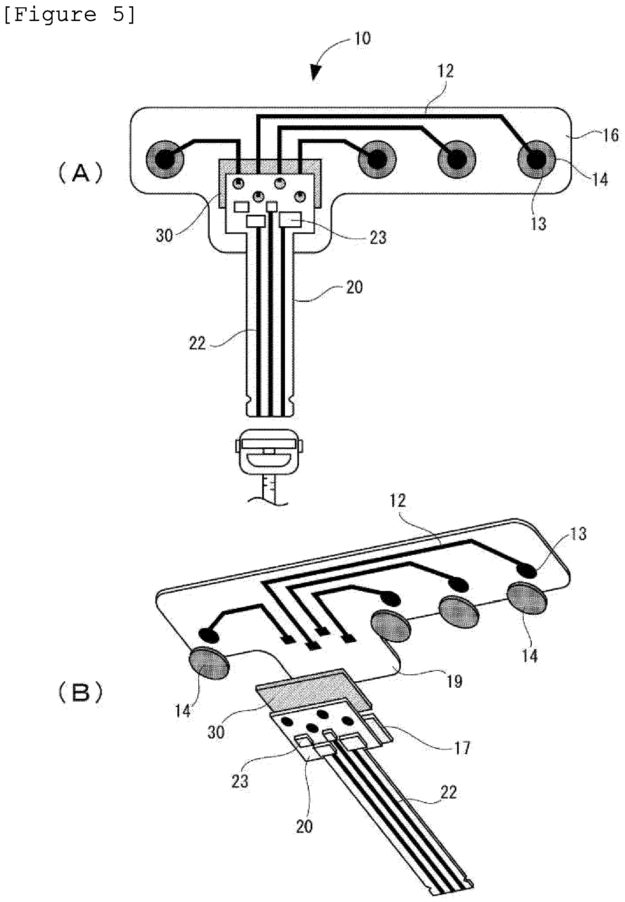

A bioelectrode 10 according to a fourth embodiment will be described with reference to Fig. 5. In the bioelectrode 10 according to the present embodiment, a connection portion for connecting a biological signal transmission/reception device that acquires an electric signal from the bioelectrode 10 or outputs an electric signal to the bioelectrode 10, or a cable extending from the biological signal transmission/reception device is provided on an electronic circuit board 20.

That is, in the bioelectrode 10 according to the present embodiment, an electrode portion 13 and a biological signal wiring portion 12 are provided on a base material 16, and the electronic circuit board 20 on which an electronic component 23 is mounted is electrically connected to the biological signal wiring portion 12 by using an anisotropic conductive paste material 30. The biological signal wiring portion 12 is provided also on the electronic circuit board 20, and the electronic circuit board 20 is electrically connected to the biological signal wiring portion 12 provided on the board by using the anisotropic conductive paste material 30. In addition, a board wiring portion 22 connected to the electronic component 23 is provided on the electronic circuit board 20. Then, the connection portion for connecting the biological signal transmission/reception device or the cable extending from the biological signal transmission/reception device is provided at an end portion of the electronic circuit board 20.

In the bioelectrode 10 formed in this manner, since various wiring portions and terminals are present on the electronic circuit board 20 on which various electronic components 23 are mounted, the electrode portion 13 and the wiring portions can be formed on the base material 16, and the electronic circuit board 20 can be electrically connected to the wiring portions by using the anisotropic conductive paste material 30. Thus, it is not necessary to provide the connection portion or the board wiring portion 22 on the base material 16.

Note that the wiring portions provided on the electronic circuit board 20 may be the board wiring portions 22 all passing through the electronic components 23, or may be the biological signal wiring portions 12 all connected to the biological signal wiring portions 12 provided on the base material 16. The through-hole 31 can be used for electrical connection between the wiring portion or the electrode portion 13 of the base material 16 and a circuit portion of the electronic circuit board 20. Furthermore, one or more electronic circuit boards 20 can be provided on the living-body-side surface and/or the back side surface of the base material 16. The base material 16 and the electronic circuit board 20 can be integrated by the adhesive or the adhesive member 17 as described above.

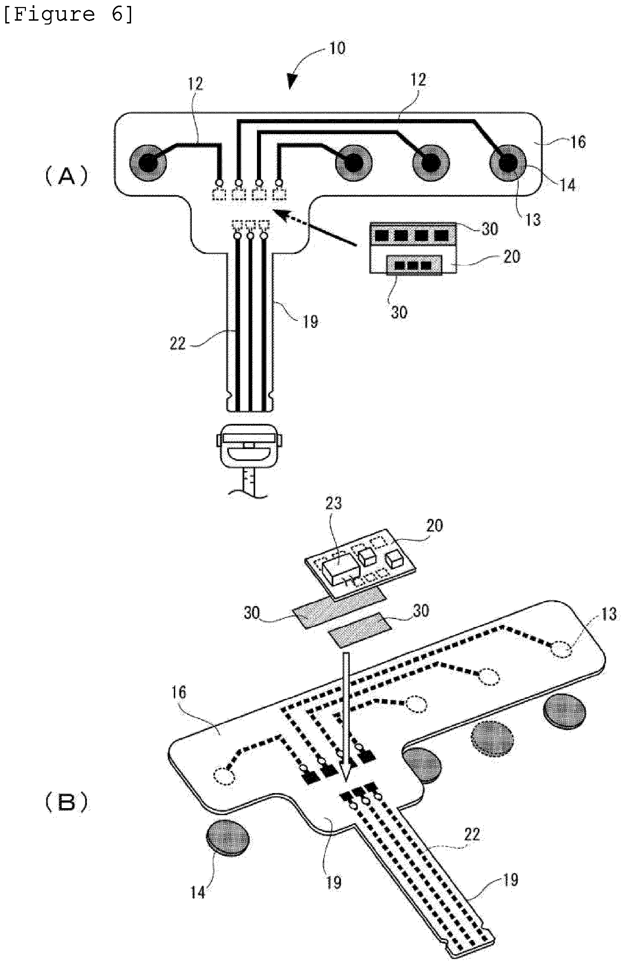

A bioelectrode 10 according to a fifth embodiment will be described with reference to Fig. 6. In the bioelectrode 10 according to the present embodiment, a biological signal wiring portion 12 and a board wiring portion 22 are electrically connected by the electronic circuit board 20.

That is, in the bioelectrode 10 according to the present embodiment, the board is elongated and forms an extending portion 19 extending to one side in the width direction. Then, a plurality of electrode portions 13 are arranged in a length direction of the base material 16, and the biological signal wiring portion 12 connected to each electrode portion 13 is provided. The board wiring portion 22 is provided on the extending portion 19, and a connection portion for connecting the biological signal transmission/reception device or the cable extending from the biological signal transmission/reception device is provided on a distal end side of the extending portion 19. The biological signal wiring portion 12 and the board wiring portion 22 are electrically connected in the electronic circuit board 20. In the present embodiment, the electronic circuit board 20 is electrically connected to each of the biological signal wiring portion 12 and the board wiring portion 22 by using an anisotropic conductive paste material 30.

The biological signal wiring portion 12 provided on the base material 16 can be connected to the board wiring portion 22 without passing through an electronic component 23 mounted on the electronic circuit board 20, or any one or all of the biological signal wiring portions 12 can be configured to be electrically connected to the board wiring portion 22 via the electronic component 23.

Also in the bioelectrode 10 according to the present embodiment, one or more electronic circuit boards 20 can be provided on a living-body-side surface and/or a back side surface of the base material 16. Further, the base material 16 and the electronic circuit board 20 can be integrated by the adhesive or the adhesive member 17 as described above. Further, the through-hole 31 can be used for electrical connection between the wiring portion or the electrode portion 13 of the base material 16 and a circuit portion of the electronic circuit board 20.

The bioelectrodes 10 according to some embodiments described above can each be used as a biological signal transmission/reception system including a biological signal transmission/reception device that acquires an electric signal from the bioelectrode 10 or outputs an electric signal to the bioelectrode 10. In addition to the above-described configuration, a known structure can be adopted for the connection between the bioelectrode 10 and the connection portion for connecting the biological signal transmission/reception device or the cable extending from the biological signal transmission/reception device.

Such a biological signal transmission/reception system can be configured to transmit an electric signal or acquire and process an electric signal from the electronic component 23 mounted on the electronic circuit board 20 provided in the bioelectrode 10 according to the above-described embodiment under the control of the biological signal transmission/reception device.

### Industrial Applicability

The bioelectrode of the present invention can be used to transmit or receive an electric signal from a living body, an electric signal for performing a low-frequency treatment on a body part, or an electric signal for performing an iontophoresis treatment. Such a bioelectrode can be variously used to acquire a biological signal or transmit a signal to a living body in the fields of medical care, beauty care, and exercise.

### Reference Signs List

- 10: Bioelectrode
- 11: Biological signal terminal
- 12: Biological signal wiring portion
- 13: Electrode portion
- 14: Gel electrolyte member
- 15: Cover sheet
- 16: Base material
- 17: Adhesive member
- 18: Opening
- 19: Bulging portion (extending portion)
- 20: Electronic circuit board
- 21: Board terminal
- 22: Board wiring portion
- 23: Electronic component
- 30: Anisotropic conductive paste material

## Claims

1. A bioelectrode (10) including an electronic circuit board (20), the bioelectrode (10) comprising:
a film-like or sheet-like flexible base material (16); and
one or more electrode portions (13) and/or wiring portions (12, 22) provided on the base material (16),
wherein the electronic circuit board (20) formed separately from the base material (16) is provided on the base material (16), and
at least any one electrode portion (13) and/or wiring portion (12, 22) provided on the base material (16) and a circuit provided on the electronic circuit board (20) are electrically connected by an anisotropic conductive paste material (30), and the electronic circuit board (20) is formed of a rigid circuit board and is bonded to the flexible base material (16) by using an adhesive member (17) such as an adhesive or a double-sided tape.

2. The bioelectrode (10) including an electronic circuit board (20) according to claim 1, wherein a through-hole (31) is provided in at least one of at least any one electrode portion (13) and/or wiring portion (12, 22) provided on the base material (16) or a circuit portion provided on the electronic circuit board (20), and the through-hole (31) is connected to the anisotropic conductive paste material (30).

3. The bioelectrode (10) including an electronic circuit board (20) according to claim 1 or 2, wherein the electrode portion (13) that acquires an electric signal from a living body is provided on a distal end side of the wiring portion (12, 22), and
the electric signal acquired from the living body by the electrode portion (13) is input to the circuit provided on the electronic circuit board (20).

4. The bioelectrode (10) including an electronic circuit board (20) according to any one of claims 1 to 3, wherein at least one of an integrated circuit, a sensor, or an amplifier is provided on the electronic circuit board (20) .

5. The bioelectrode (10) according to any one of claims 1 to 4, wherein the sensor is a sensor that comes into contact with the living body, such as a thermistor or a pH sensor, and is provided on a surface of the base material (16) on the same side as the electrode portion (13) that is provided on the distal end side of the wiring portion (12, 22) and acquires the electric signal from the living body.

6. The bioelectrode (10) including an electronic circuit board (20) according to any one of claims 1 to 5, wherein the electrode portion (13) and/or the wiring portion (12, 22) provided on the base material (16) transmits or receives the electric signal from the living body, an electric signal for performing a low-frequency treatment on a body part, or an electric signal for performing an iontophoresis treatment.

7. A biological signal transmission/reception system comprising:
the bioelectrode (10) according to any one of claims 1 to 6; and
a biological signal transmission/reception device that acquires an electric signal from the bioelectrode (10) or outputs an electric signal to the bioelectrode (10).

8. A method for manufacturing a bioelectrode (10) including an electronic circuit board (20), the method comprising:
a conductive material application step of applying a conductive material on a film-like or sheet-like flexible base material (16) to form an electrode portion (13) and/or a wiring portion (12, 22);
a layering step of layering an anisotropic conductive paste material (30) or an anisotropic conductive film material on at least any one electrode portion (13) and/or wiring portion (12, 22) formed by the application, and layering the electronic circuit board (20) formed separately from the base material (16) on the anisotropic conductive paste material (30); and
a thermocompression bonding step of electrically connecting the electrode portion (13) and/or the wiring portion (12, 22), the anisotropic conductive paste material (30) or the anisotropic conductive film material, and the electronic circuit board (20) by thermocompression bonding.
